## Europäisches Patentamt

(19) **European Patent Office**

**Office européen des brevets**

(11) Numéro de publication: **0 112 574**
**A1**

(12) # DEMANDE DE BREVET EUROPEEN

(21) Numéro de dépôt: 83113098.4

(22) Date de dépôt: 24.12.83

(51) Int. Cl.³: **A 61 M 5/28,** A 61 M 5/315, B 65 D 81/32

(30) Priorité: 27.12.82 PC /CH82/001 39

(43) Date de publication de la demande: 04.07.84 Bulletin 84/27

(84) Etats contractants désignés: **AT BE CH DE FR GB IT LI LU NL SE**

(71) Demandeur: **MEDITEC S.A., 11, Boulevard du Prince Henri, L-2014 Luxembourg (LU)**

(72) Inventeur: **Meyer, Gabriel, 10 A, Chemin des Princes, CH-1222 Vesenaz (CH)**
Inventeur: **Howald, Ernst, 10 B, Chemin des Princes, CH-1222 Vesenaz (CH)**

(74) Mandataire: **Nithardt, Roland, CABINET ROLAND NITHARDT Rue Edouard Verdan 15, CH-1400 Yverdon (CH)**

(54) **Seringue préremplie à double compartiment.**

(57) La seringue préremplie selon l'invention comporte un corps de seringue (10) sensiblement cylindrique pourvu d'un col (11) dont l'extrémité est équipée d'un bourrelet périphérique (12) et d'un rebord (13) en forme d'ailettes de préhension ménagées le long de son extrémité postérieure. Ce corps de seringue contient un piston intérieur (14) muni d'une soupape bidirectionnelle (15) et divisant la cavité intérieure au corps de la seringue en une première chambre (16) contenant par exemple un produit pulvérulent (17) et un gaz inerte détendu (18) et une seconde chambre (19) contenant un produit liquide (20). L'extrémité postérieure du corps de la seringue (10) est obturée par un piston (21) couplé à une tige de piston (22) pourvue d'un poussoir (23). Le col (11) du corps de la seringue (10) est obturé par un organe d'obturation (24) équipé d'un conduit d'écoulement intérieur en forme de T et mobile dans le sens de la double flèche A entre une position d'obturation et une position d'écoulement.

ACTORUM AG

## SERINGUE PREREMPLIE A DOUBLE COMPARTIMENT

La présente invention concerne une seringue préremplie à double compartiment comportant un corps de seringue ainsi qu'un piston intérieur muni d'une soupape et divisant le corps de seringue en deux chambres contenant respectivement un produit liquide ou pulvérulent, et un produit liquide destiné à être mélangé audit produit liquide ou pulvérulent à l'intérieur de la chambre correspondante.

On connait déjà des seringues dites seringues mélangeuses comportant un piston intérieur divisant le corps de la seringue en deux chambres dont l'une délimitée par ledit piston intérieur et le fond de ce corps de seringue contient une poudre, et dont l'autre, délimitée par ledit piston intérieur et un second piston muni d'une tige de piston, contient un liquide prévu pour dissoudre cette poudre. Un tel système est décrit par le brevet américain No 3'380'451. Le piston intérieur est pourvu d'une soupape unidirectionnelle qui se présente sous la forme d'une languette s'ouvrant théoriquement lorsque la pression engendrée dans la chambre contenant le liquide, suite à une pression exercée sur la tige du piston, est supérieure à la pression régnant dans la chambre contenant la poudre. Si le piston intérieur n'est pas équipé d'organes de retenue suffisammment efficaces pour assurer un blocage en position de ce piston, la languette qui constitue la soupape doit céder à une surpression très faible, ce qui est incompatible avec les conditions d'une séparation efficace et étanche des deux chambres pendant le stockage.

Dans ce cas également, le piston a tendance à avancer chaque fois que l'opérateur exerce une pression sur la tige du second piston, ce qui a pour effet de réduire la pression différentielle et par conséquent de limiter le volume de liquide passant par cette soupape unidirectionnelle de la chambre correspondante dans celle contenant la poudre. Le nombre des petites pressions à exercer sur le second piston pour effectuer le mélange liquide-poudre est relativement grand, ce qui constitue un des inconvénients majeurs qui sont à l'origine de l'échec commercial de ce système. Un autre inconvénient provient de l'absence de garantie quant à l'étanchéité entre les deux chambres.

Si l'on suppose que la languette du piston intérieur est suffisamment hermétique, c'est-à-dire qu'elle ne se vide que lorsque la pression différentielle exercée sur elle dépasse un certain seuil prédéterminé relativement élevé, ledit piston intérieur doit obligatoirement être équipé d'organes de retenue pour éviter que celui-ci se déplace lorsque l'opérateur exerce une poussée sur le second piston, ce qui a pour effet de résorber au moins en partie la pression différentielle nécessaire à l'ouverture de la languette. Le problème des organes de retenue dont la réalisation s'avère critique n'est pas abordé dans la description de ce système. En outre, la présence d'organes de retenue est extrêmement gênante à l'injection puisque l'infirmière doit déployer une force suffisante pour vaincre la résistance due à ces organes, au moment de l'injection, ce qui entraîne une injection irrégulière par à-coups, et qui est contraire aux normes actuellement en vigueur.

Si l'on souhaite obtenir un écoulement rapide du liquide de la seconde vers la première chambre, en un nombre réduit d'opérations, la chambre contenant la poudre doit avoir un volume important, ce qui est contraire aux exigences en matière d'encombrement et de coût qui tendent plutôt à favoriser une construction pour laquelle la chambre de la poudre présente un volume restreint.

Quel que soit le mode d'utilisation de ce système, il présente des inconvénients importants qui le rendent difficilement utilisable en pratique.

Un autre dispositif connu est décrit par le brevet français No 1.574.830. Comme précédemment, la seringue comporte deux chambres séparées par un piston intérieur, pourvu d'une soupape unidirectionnelle, et des organes de retenue agencés le long de la paroi intérieure du corps de la seringue et destinés à retenir le piston intérieur lorsqu'une surpression est exercée sur le liquide contenu dans l'une des chambres. Grâce à ces organes de retenue, le piston intérieur reste fixe en position, dans un premier temps, de sorte qu'il se crée une pression différentielle entre les deux chambres, permettant l'ouverture de la soupape unidirectionnelle. Du

3

0112574

fait de l'écoulement du liquide de la chambre postérieure vers la chambre antérieure, la pression croît dans cette dernière. A condition que les organes de retenue soient assez forts pour maintenir le piston intérieur en position, la pression différentielle diminue progressivement et proportionnellement à la diminution du volume occupé par le gaz comprimé dans la chambre antérieure contenant à l'origine la poudre et servant de chambre de mélange. La diminution progressive de la pression différentielle a pour effet de s'opposer à une ouverture normale de la soupape unidirectionnelle. Il en résulte une diminution de débit malgré une augmentation de la pression exercée sur la tige du second piston. Pour permettre d'obtenir un débit normal il convient d'augmenter considérablement la pression exercée sur le piston extérieur, pression qui se transmet intégralement sur le piston intérieur tant que les organes de retenue parviennent à retenir ce dernier en position. Il en résulte que les organes de retenue doivent être suffisamment puissants pour assurer le maintien en position du piston intérieur malgré une pression importante exercée sur le piston extérieur. Si ces organes de retenue cèdent prématurément, le système se bloque, de sorte que le mélange ne peut pas se faire de manière complète.

Selon une forme de réalisation, les organes de retenue sont constitués par au moins un bourrelet ou une gorge ménagée le long de la paroi intérieure du corps de la seringue. Les deux solutions sont difficiles à réaliser et coûteuses. En outre, ils imposent un prépositionnement très précis du piston intérieur de sorte qu'il devient indispensable d'adapter le modèle en fonction des produits et des volumes de ces produits à injecter, et empêchent l'utilisation d'une seringue standard utilisable pour une gamme étendue de produits et de volumes.

Un autre inconvénient de ce système provient de ce qu'il est indispensable de dégazer la chambre antérieure contenant la poudre au moment du remplissage, ce qui risque d'entraîner une partie de cette poudre au cours de cette opération de dégazage.

Par ailleurs, si l'on analyse le cycle de fonctionnement de ce système, on constate que les risques d'un blocage prématuré sont importants, et qu'il n'est guère certain que le cycle de mélange poudre-liquide soit complet. En effet, pour procéder au mélange, il est indispensable d'exercer une pression sur la tige du piston extérieur, pression qui se transmet intégralement au piston intérieur, qui prend appui contre l'organe de retenue constitué par un bourrelet ou une gorge empêchant un déplacement axial dudit piston intérieur. Si une partie du liquide contenu dans la chambre postérieure s'écoule à travers la vanne unidirectionnelle dans la chambre antérieure contenant la poudre, le gaz, également contenu dans cette chambre est comprimé en raison de la réduction du volume qu'il subit. Lorsque l'opérateur relâche la pression exercée sur le piston extérieur, la soupape unidirectionnelle se referme et le gaz comprimé de la chambre antérieure a tendance à se détendre en refoulant vers l'arrière le piston intérieur. De ce fait, ce piston s'éloigne des organes de retenue qui sont indispensables à la création d'une pression diférentielle entre les deux chambres, elles-mêmes à l'origine de l'ouverture de la soupape unidirectionnelle. Une nouvelle pression exercée sur le piston extérieur n'aura pour seul effet que de déplacer axialement le piston intérieur et de l'amener en contact avec ces organes de retenue solidaires du corps de la seringue. De ce fait, ce système perd l'avantage d'une réduction espérée du volume de la chambre antérieure. Bien au contraire, pour pouvoir fonctionner efficacement, ce système doit comporter une chambre antérieure plus grande que le volume de la chambre postérieure, afin de permettre un mélange en un seul cycle. En pratique, la chambre antérieure peut avoir un volume correspondant à la somme des volumes des composants initialement contenus dans la première et la seconde chambre du corps de la seringue. Le volume de la chambre antérieure qui constitue également la chambre du mélange définit avec précision la position des organes de retenue.

En résumé, les inconvénients de ce système proviennent de ce que les bourrelets ou gorges ménagées le long des parois intérieures du corps de la seringue sont difficiles à réaliser et coûteux, de ce que les organes de retenue constitués par ces bourrelets ou gorges provoquent des à-coups lors de l'injection, de ce que ces organes de retenue doivent être positionnés en un emplacement précis, ce qui pose des problèmes au remplissage et ne permet aucune souplesse d'adaptation à des produits et des volumes différents, et en ce que l'obligation de disposer d'une chambre antérieure de volume important engendre un encombrement important désagréable au stockage et provoque une perte de maniabilité de la seringue lors de son utilisation.

La multiplication des organes de retenue disposés à intervalles réguliers les uns des autres ne pourrait qu'aggraver les inconvénients mentionnés ci-dessus.

Selon une forme de réalisation particulière, le piston intérieur est pourvu d'une lamelle périphérique dont le but consiste à remplacer les organes de retenue en assurant un freinage tendant à empêcher la progression axiale du piston intérieur en direction de la chambre antérieure. Une telle lamelle offre obligatoirement une certaine résistance au recul, si l'on veut qu'elle soit en mesure de remplacer efficacement les organes de retenue décrits précédemment. En effet, l'on sait que l'organe de retenue doit être puissant, en particulier lorsque la pression différentielle diminue, car la limite de décrochement de l'organe de retenue dépend de la pression exercée par le liquide, totalement incompressible, de la chambre antérieure. Pour engendrer une pression différentielle susceptible de maintenir ouverte la soupape unidirectionnelle, il est nécessaire d'accroître la pression exercée sur le piston extérieur, ce qui risque bien de provoquer le relâchement de la languette de retenue et le blocage du système. Il en résulte que la languette de retenue doit être conçue de manière à assurer un freinage puissant. Les conséquences négatives en sont que l'injection se fait par à-coups et nécessite l'exercice d'une pression importante sur le piston extérieur, et que le mélange ne peut se faire qu'au cours de plusieurs cycles, ce qui ralentit l'opération et accroît désagréablement le nombre des opérations préliminaires à l'injection.

Les autres formes de réalisation comportent soit une manchette, soit des lèvres d'étanchéité très minces ne permettant pas non plus de pallier les divers inconvénients décrits. La manchette ne peut fonctionner que si la pression de la chambre postérieure est supérieure à celle de la chambre antérieure. Pour être efficace, elle doit exercer un effet de freinage désagréable à l'injection. Les lèvres qui se replient théoriquement vers l'arrière au moment de l'injection pour éviter un freinage lors de cette opération, engendrent des problèmes de mise en place délicats à résoudre. La finesse des lèvres réversibles semble tout à fait incompatible avec la nécesité de disposer d'organes de retenue puissants. En outre, si l'on exerce sur le piston une pression suffisante pour engendrer une pression différentielle entre les deux chambres, cette pression étant assez élevée pour assurer l'ouverture de la soupape unidirectionnelle, les lèvres minces auront nécesairement tendance à se retrousser ce qui, si cela se produit, bloque le système. Le déblocage nécessite un mouvement de recul du piston extérieur pour créer un vide partiel qui engendre un rappel desdites lèvres minces. La présence de ces lèvres oblige l'opérateur à effectuer plusieurs mouvements de va-et-vient. La mise en place du piston intérieur nécessite une précision relativement importante.

Tous ces systèmes présentent en fait l'inconvénient de comporter des soupapes unidirectionnelles devant répondre à des exigences contradictoires. La garantie d'une bonne étanchéité entre les deux chambres et la facilité d'ouverture de la soupape sous faible pression constituent deux contraintes contradictoires.

La présente invention se propose de pallier les inconvénients susmentionnés en réalisant un dispositif exempt d'organes de retenue, gênant à l'injection, qui permet un transvasement rapide du liquide de la seconde dans la première chambre dans un milieu fermé et compact et une injection sans à-coups du mélange dans le tissu cutané du patient.

Dans ce but, la seringue préremplie à double compartiment selon l'invention est caractérisée en ce que le piston intérieur comporte un évidement central dans lequel est logée ladite soupape, en ce que cette soupape est mobile axialement dans cet évidement entre une première position dite position d'obturation et une seconde position dite position d'écoulement, en ce que la soupape présente une première surface d'appui orientée vers l'une des chambres et une seconde surface d'appui orientée vers l'autre chambre, et en ce que cette soupape comporte au moins un canal d'écoulement agencé pour faire communiquer les deux chambres entre elles pour permettre l'écoulement du liquide vers la chambre contenant ledit produit liquide ou pulvérulent lorsque la soupape se trouve dans sa position d'écoulement, ainsi que des organes ressorts agencés pour solliciter la soupape dans sa position d'obturation.

Les principaux aspects et avantages de la seringue préremplie à double compartiment selon l'invention, seront mieux compris en référence à la description de divers exemples de réalisation et du dessin annexé dans lequel :

Les figures 1 à 4 représentent des vues en coupe longitudinale d'une première forme de réalisation de la seringue préremplie à double compartiment selon l'invention, successivement dans la position de stockage, dans deux positions intermédiaires et dans la position d'injection,

Les figures 5 à 9 sont des vues en coupe longitudinale d'une autre forme de réalisation de la seringue préremplie selon l'invention, successivement dans la position de stockage, dans trois positions intermédiaires et dans la position d'injection,

Les figures 10 à 12 représentent une forme de réalisation d'une ampoule-seringue préremplie à double compartiment, illustrée successivement dans la position de stockage, dans une position intermédiaire et dans la position d'injection,

Les figures 13 et 14 représentent une forme de réalisation particulière des organes d'obturation susceptibles d'être adaptés à l'extrémité ouverte de l'ampoule-seringue telle que représentée par les figures 10 à 12, respectivement dans la position d'obturation et dans la position d'écoulement,

Les figures 15 et 16 représentent une autre forme de réalisation de l'organe d'obturation susceptible d'être adapté sur les ampoules- seringues des figures 10 à 12, ces organes étant représentés respectivement dans leur position d'obturation et dans leur position d'utilisation,

Les figures 17 et 18 représentent une forme de réalisation particulière du piston intérieur et de sa soupape, tels que montés à l'intérieur d'une quelconque des seringues préremplies à double compartiment selon l'invention, les soupapes étant respectivement présentées en position d'obturation et en position d'écoulement,

Les figures 19 et 20 représentent une autre forme de réalisation du piston intérieur, la soupape étant respectivement présentée en position fermée et en position ouverte,

Les figures 21 et 22 représentent une variante de l'exécution illustrée par les figures 19 et 20, la soupape étant respectivement illustrée en position fermée et en position ouverte,

Les figures 23 à 26 illustrent un autre mode d'exécution de la seringue préremplie à double compartiment selon l'invention, respectivement dans sa position de stockage, deux positions intermédiaires et dans sa position d'utilisation,

Les figures 27 et 28 illustrent deux états d'une variante d'une ampoule-seringue à deux compartiments selon l'invention, et

La figure 29 illustre le piston intérieur de l'ampoule-seringue des figures 27 et 28 en position ouverte.

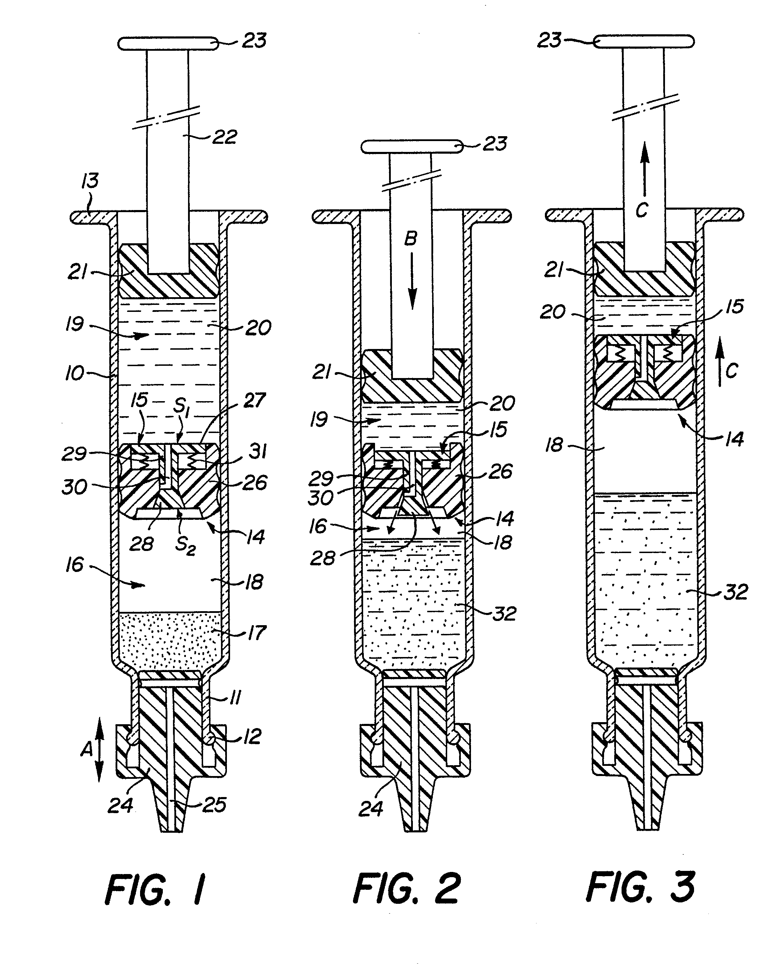

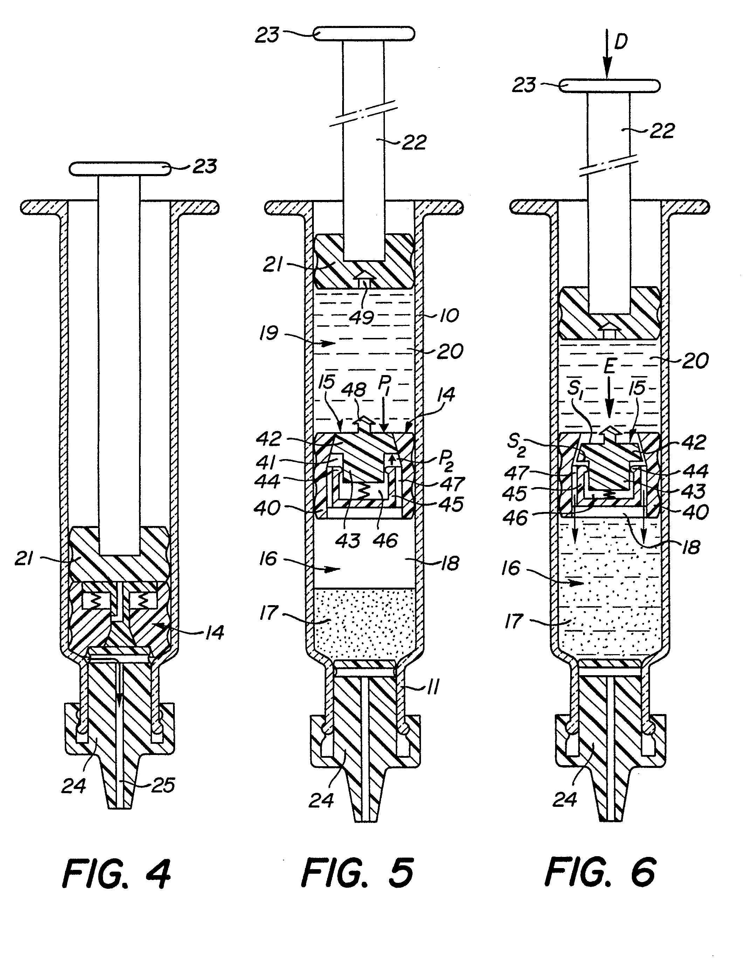

En référence aux figures 1 à 4, la seringue préremplie selon l'invention comporte un corps de seringue 10 sensiblement cylindrique pourvu d'un col 11 dont l'extrémité est équipée d'un bourrelet périphérique 12 et d'un rebord 13 en forme d'ailettes de préhension ménagées le long de son extrémité postérieure. Ce corps de seringue contient un piston intérieur 14 muni d'une soupape bidirectionnelle 15 et divisant la cavité intérieure au corps de la seringue en une première chambre 16 contenant par exemple un produit pulvérulent 17 et un gaz inerte détendu 18 et en une seconde chambre 19 contenant un produit liquide 20. L'extrémité postérieure du corps de la seringue 10 est obturée par un piston 21 couplé à une tige de piston 22 pourvue d'un poussoir 23. Le col 11 du corps de la seringue 10 est obturé par un organe d'obturation 24 équipé d'un conduit d'écoulement intérieur en forme de T et mobile dans le sens de la double flèche A entre une position d'obturation illustrée par la fig. 1 et une position d'écoulement illustrée par la fig. 4.

Le piston intérieur 14 se compose d'un corps 26 en élastomère sensiblement cylindrique comportant de préférence des bourrelets périphériques pour garantir l'étanchéité entre les deux chambres 16 et 19. Dans ce corps est ménagée une cavité dont la forme est adaptée à celle de la soupape 15. Cette soupape se compose dans l'exemple décrit d'une plaque circulaire 27 de surface S1 orientée vers l'intérieur de la seconde chambre 19, d'un bloc tronconique 28 dont la surface extérieure correspondant à la grande base a une surface S2 orientée vers l'intérieur de la première chambre et d'un tronçon cylindrique 29 qui assure la liaison entre la plaque 27 et le bloc 28. Un conduit 30 en forme de L débouche d'une part dans la seconde chambre 19 et d'autre part contre la paroi de l'alésage cylindrique ménagé dans le corps 26 du piston 14. Des organes ressort 31 sont disposées dans la cavité intérieure au corps 26 du piston intérieur 14 pour solliciter la soupape 15 dans la position illustrée par la fig.1.

La fig. 2 illustre une position intermédiaire dans laquelle l'opérateur exerce une poussée sur le piston 21 dans le sens de la flèche B en agissant sur le poussoir 23 de manière à augmenter la pression règnant dans les deux chambres 16 et 19. Ceci engendre un déplacement vers le bas (sur la fig. 2) du piston intérieur 14 étant donné qu'il n'existe aucun organe de retenue pour bloquer ce piston en position. Etant donné que la chambre 19 est entièrement remplie d'un liquide 20 la pression exercée par le piston 21 se transmet intégralement au piston intérieur 14 qui transmet cette pression au contenu de la chambre 16 en comprimant le gaz 18. En raison de la construction particulière de la soupape 15 comportant comme mentionné ci-dessus un plateau supérieur de surface S1 correspondant par exemple à 80% de la surface supérieure du piston intérieur et un plateau inférieur dont la surface S2 représente par exemple le 20% de la surface totale de la section du piston intérieur, la soupape s'ouvre et permet au liquide 20 de s'échapper à travers le conduit 29 et entre le bloc tronconique 28 et son siège conique, pour s'écouler dans la chambre 16 et se mélanger à la poudre 17. Pendant toute l'opération de décharge du liquide 20 vers la chambre 16, la soupape reste ouverte, étant donné que les conditions ayant provoqué son ouverture restent inchangées pendant un certain temps. Pendant tout ce temps, la pression dans les deux chambres augmente proportionnellement à la réduction du volume que peut occuper le gaz dans la chambre 16, jusqu'à une valeur maximale de cette pression correspondant au stade d'incompressibilité du gaz. Lorsque cette incompressibilité est atteinte, l'opérateur relâche la pression sur le poussoir 23, ce qui a pour effet de provoquer une détente du gaz 18, une fermeture de la soupape 15, une remontée du piston intérieur 14 dans le sens de la flèche C (voir fig. 3) et une remontée identique du piston 21. Une nouvelle pression exercée sur le poussoir 23 permet de recommencer à nouveau l'opération tendant à faire passer le liquide restant 20, dans la chambre antérieure 16 contenant à présent le mélange 32 et le gaz 18. Selon les volumes pris en considération, le mélange pourra s'effectuer en un seul ou en plusieurs cycles successifs jusqu'au moment où le piston 14 soit remonté de manière à adopter une position accolée ou éventuellement encliquetée dans le piston 21. Le mélange étant effectué la seringue est prête à l'injection. A ce moment l'opérateur retourne la seringue et repousse l'obturateur 24 dans sa position illustrée par la fig. 4 de manière à libérer les orifices du canal radial du conduit 25, procède au dégazage préalable de la seringue et effectue l'injection du mélange. En deux ou trois pressions rapides successives, il est possible de faire passer approximativement dix ml de liquide de la chambre 19 dans la chambre 16 qui avait à l'origine une capacité de l'ordre de 3 à 4 ml et qui contient finalement un mélange dont le volume est de l'ordre de 13 à 14 ml, le volume de la chambre 19 étant réduit à zéro.

L'absence d'organes de retenue permet d'assurer un mélange très rapide des deux composants et garantit une grande souplesse à l'injection. Du fait que le volume de la première chambre augmente pendant que l'on effectue le mélange alors que celui de la seconde chambre diminue simultanément, il n'est pas nécessaire de prévoir pendant le stockage une chambre de mélange dont le volume est au moins égal à la somme des volumes des deux composants, ce qui permet de réduire considérablement l'encombrement de la seringue et faciliter son stockage.

L'utilisation de cette seringue mélangeuse est particulièrement confortable du fait que l'opérateur n'a à exercer qu'un seul mouvement qui consiste à presser sur le poussoir 23 et à relâcher ce poussoir sans effectuer une quelconque manipulation à l'avant de la seringue.

Lors du montage aucun positionnement du piston intérieur n'est imposé. Si la première chambre est déjà remplie il n'est pas nécessaire d'effectuer un dégazage, car il suffit d'introduire le piston en appuyant sur la soupape 15 pour permettre au gaz de s'échapper.

Lors de la mise en place du piston extérieur 21, il se produit inévitablement une légère surpression dans la chambre 19 étant donné que le piston extérieur 21 est en appui contre la surface du liquide contenu dans cette chambre. Contrairement au système connu où cette surpression peut éventuellement engendrer l'ouverture de la soupape, si les organes de retenue sont suffisamment puissant pour bloquer le piston intérieur en position, une légère surpression est sans risque dans le dispositif décrit grâce au libre coulissement du piston intérieur. La force de poussée des organes ressorts 31 est déterminée pour qu'une telle surpression ne puisse en aucune manière ouvrir la soupape 15 mais soit compensée par un léger déplacement axial du piston intérieur 14, tendant à augmenter quelque peu la pression du gaz 18.

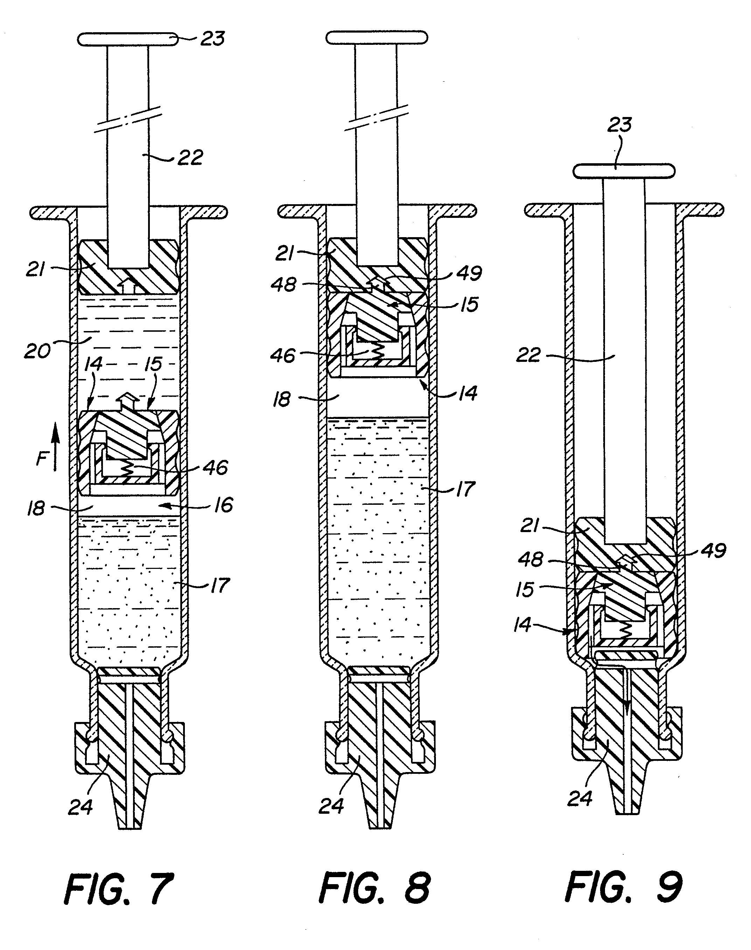

Les figures 5 à 9 illustrent une autre forme de réalisation de la seringue décrite en référence aux figures 1 à 4. Pour faciliter la description de cette réalisation, on utilisera les mêmes références que précédemment pour les éléments identiques. La seringue représentée comporte comme précédemment un corps de seringue 10, un piston intérieur 14 muni d'une soupape 15 qui divise le corps de la seringue 10 en deux chambres 16 et 19, un second piston 21 équipé d'une tige de piston 2, d'un poussoir 23 et un organe d'obturation 24 adapté sur le col 11 du corps de la seringue 10. Le piston intérieur 14 se compose d'un corps sensiblement cylindrique 40 pourvu d'un évidement central 41 dans lequel est logée la soupape 15. Cette soupape se compose d'une partie supérieure tronconique 42 solidaire d'un talon cylindrique 43 dont la périphérie se trouve en appui contre un bourrelet 44 qui entoure le bord supérieur d'une partie centrale en forme de cuvette 45 du corps 40 du piston intérieur. Cette cuvette 45 contient un organe ressort 46 qui tend à repousser la soupape 15 dans sa position illustrée par la fig. 5. Des conduits axiaux 47 sont ménagés dans l'épaisseur du corps parallèlement aux parois latérales de la cuvette 45. La surface supérieure de la soupape 15 comporte un organe protubérant 48 dont le profil est complémentaire à celui d'un évidement 49 ménagé à la base du piston 21, ces deux organes étant destinés à permettre l'accouplement du piston intérieur 14 et du piston 21.

La fig. 5 représente la position de stockage c'est-à-dire la position dans laquelle la première chambre contient une poudre ou un liquide 17 surmonté d'un gaz 18 et où la seconde chambre 19 contient un liquide 20. Aucune pression n'est exercée sur le piston 21 et la soupape 15 est fermée sous l'effet du ressort 46. Le dispositif de la fig. 6 représente une position intermédiaire de cette seringue. Une poussée est exercée sur le poussoir 23 dans le sens de la flèche D, ce qui a pour effet, après un léger déplacement préalable du piston intérieur non retenu par des organes de retenue, d'ouvrir la soupape 15 et de permettre l'écoulement du liquide 20 entre l'élément tronconique 42 de cette soupape et son siège conique ménagé dans le corps cylindrique 40 du piston intérieur, et à travers les conduits 47. Le ressort 46 est comprimé. Le bourrelet 44 assure l'étanchéité entre la cuvette 45 et le talon cylindrique 43 de la soupape 15. De ce fait la surface S2 est représentée par la surface annulaire de l'épaulement qui sépare la partie tronconique et le talon cylindrique de la soupape 15, la surface S1 étant représentée par la surface de la soupape 15 se trouvant en contact avec le liquide 20.

Comme précédemment, au cours d'une première opération une partie du liquide 20 passe dans la première chambre pour se mélanger au produit qu'elle contient. Si l'opérateur relâche le poussoir 23, le piston intérieur 14 et le piston supérieur 21 remontent dans le sens de la flèche F comme le montre la fig. 3. Après deux ou trois cycles d'appui sur le poussoir, le piston intérieur 14 vient s'accoupler au piston 21. Après un dégazage préalable de la chambre de mélange et une ouverture de l'organe d'obturation 24, le mélange peut être injecté dans le tissu du patient.

Les réalisations décrites ci-dessus fonctionnent en pratique d'une manière tout à fait similaire. En exerçant une pression sur la tige du piston extérieur, cette pression se transmet au liquide de la chambre postérieure d'une manière uniforme selon le principe de Pascal. Cette pression se transmet intégralement sur la surface supérieure du piston intérieur. Etant donné la construction particulière de ce piston intérieur, la pression exercée sur le piston extérieur s'exerce par exemple à 80% sur le plateau d'appui supérieur de la soupape (la surface S1 représentant environ 80% de la surface totale de la section du piston intérieur) et à raison de 20% sur la couronne restante du piston intérieur. En raison de la très faible résistance de frottement exercée par le piston intérieur contre les parois du corps de la soupape, ce piston intérieur à tendance à avancer en direction de la chambre antérieure, au cours d'un premier temps. De ce fait, la pression règnant dans la chambre postérieure se transmet intégralement au contenu de la chambre antérieure et provoque la compression du gaz qui surmonte la substance liquide ou pulvérulente contenue dans cette chambre antérieure. Compte tenu de la construction particulière de la soupape, cette pression se répartit à raison de 80% sur la surface du piston proprement dit et environ 20% (qui correspondent à la surface S2) sur le plateau inférieur de cette soupape.

Tant que la soupape n'est pas ouverte, le piston intérieur continue d'avancer, ce qui a pour effet d'augmenter la pression dans tout le système proportionnellement à la compression exercée sur le gaz contenu par la chambre antérieure. Toutefois, ce déplacement est extrêmement faible, par exemple de l'ordre de quelques millimètres, car pour une pression déterminée, le ressort agissant sur la soupape cède et il se produit une sorte "d'écrasement relatif" entre le plateau supérieur de la soupape et le piston intérieur, cet écrasement provoquant l'ouverture de la soupape et par conséquent l'ouverture du canal de décharge. Dès que cette soupape est ouverte, le piston intérieur se stabilise à l'intérieur du corps de la seringue quelle que soit l'intensité de la pression exercée sur le piston extérieur. La soupape reste ouverte tant que le système est sous pression et notamment tant que cette pression est croissante. L'écoulement du liquide à travers le canal de décharge se produit jusqu'au moment où l'on atteint le stade d'incompressibilité totale du gaz contenu dans la chambre antérieure. Dans cet état la soupape reste ouverte mais l'échange de liquides ne peut plus se faire. Lorsque ce stade est atteint, il suffit de relâcher la pression exercée sur la tige de piston ce qui a pour effet de détendre le ressort agissant sur la soupape et de fermer cette dernière. En raison de l'absence de pression exercée sur le liquide de la chambre postérieure, la surpression exercée sur le gaz fortement comprimé contenu dans la chambre antérieure a pour effet de refouler le piston intérieur en direction du piston supérieur jusqu'au moment où le gaz soit entièrement dilaté. Dès que l'équilibre des pressions est rétabli entre les deux chambres, l'utilisateur peut à nouveau exercer une pression sur le poussoir du piston extérieur et enclencher un nouveau cycle opératoire.

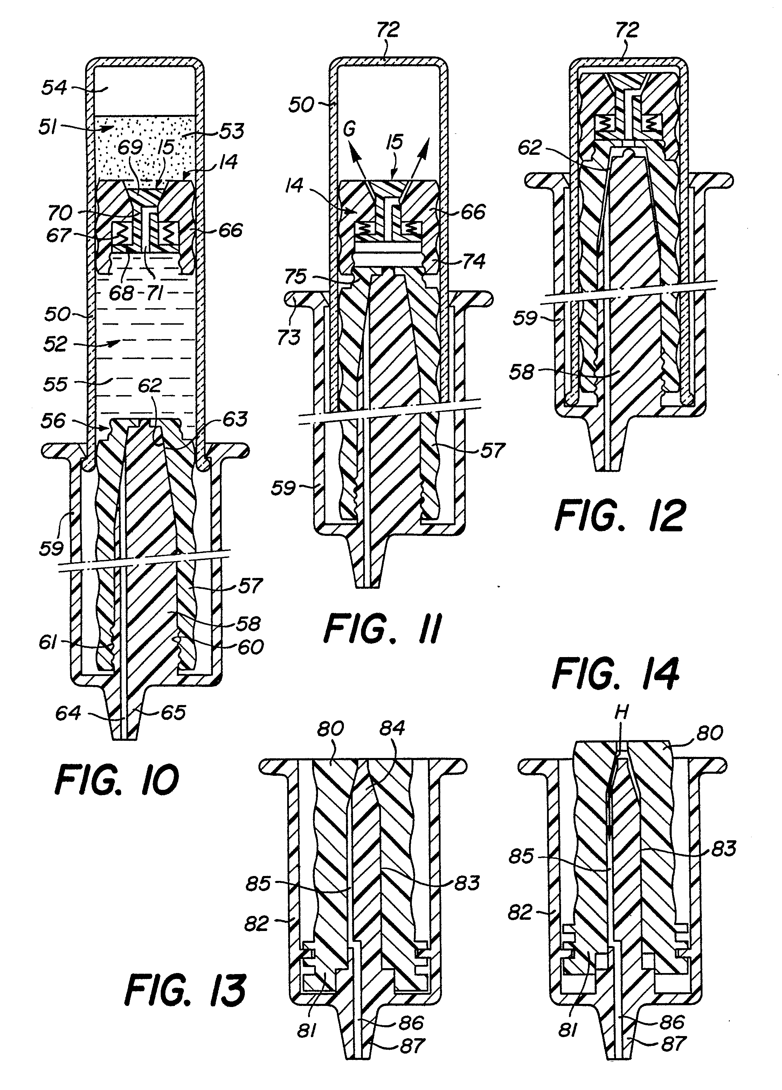

Les figures 10, 11 et 12 illustrent une autre forme de réalisation dans laquelle le corps de la seringue a été remplacé par une ampoule 50 fermée à l'une de ses extrémités. De ce fait, le second piston, qui n'a plus de raison d'être, a été supprimé. Cette ampoule est divisée en deux chambres 51 et 52 dont la première 51 contient une poudre ou un liquide 53 et un gaz inerte 54 et dont la seconde contient un liquide 55 destiné à être mélangé à la poudre 53. Comme pour les réalisations précédentes, les deux chambres sont séparées par un piston intérieur 14 muni d'une soupape 15, qui seront décrits plus en détail ci-dessous.

L'extrémité ouverte de l'ampoule 50 est obturée par un organe d'obturation 56 comportant un bouchon d'obturation 57 sensiblement cylindrique, dont le diamètre est au moins égal au diamètre du tronçon de l'ampoule 50 voisine de son extrémité ouverte, un obturateur 58 logé à l'intérieur d'un alésage central ménagé dans le bouchon 57 et une capsule 59 solidaire ou réalisé d'une pièce avec l'obturateur 58. Dans la position représentée par la fig. 10, l'obturateur 55, réalisé d'une pièce avec la capsule 59 comporte un filetage 60 qui coopère avec un filetage complémentaire 61 ménagé à la base de l'alésage central du bouchon 57 et vissé à fond dans cet alésage de telle manière que son tronçon d'extrémité 62 tronconique soit en appui contre le siège conique 63 constituant l'extrémité supérieure dudit alésage central du bouchon 57. Un conduit d'écoulement 64 aboutissant sensiblement au centre de l'embout porte-aiguille 65 de la capsule 59, prend naissance le long de la périphérie du tronçon d'extrémité tronconique 62.

Le piston intérieur 14 comporte un corps 66 sensiblement cylindrique, réalisé de préférence en un élastomère, et qui est pourvu d'un évidement central agencé pour permettre la mise en place de la soupape 15 et des organes ressorts 67. La soupape 15 est sensiblement identique à celle décrite en référence aux figures 1 à 4 et se compose d'une plaque 68 de forme circulaire, d'un élément tronconique 69 et d'un tronçon cylindrique 70 qui assure la liaison entre la plaque 68 et l'élément tronconique 69. Un conduit intérieur 71 permet l'écoulement du liquide 55 vers la chambre 51 lorsque la soupape 15 est ouverte.

La fig. 10 représente la position de stockage dans laquelle la soupape 15 et l'organe d'obturation 56 sont fermés. Le dispositif fonctionne sensiblement comme les sytèmes munis d'un piston extérieur décrits précédemment. Lorsque l'opérateur exerce une pression sur le fond 72 de l'ampoule, en appuyant par exemple à l'aide du pouce sur ce fond et en maintenant l'ensemble par les ailettes 73 de la capsule 59, il enfonce l'ampoule 50 par rapport à la capsule 59 et par rapport au bouchon 57. Ceci a pour effet de créer une augmentation de pression dans les deux chambres 51 et 52, d'ouvrir la soupape 15 et de permettre l'écoulement du liquide 55 dans le sens des flèches G. Lorsque tout le liquide 55 est passé dans la chambre de mélange, le piston intérieur 14 vient se coupler à l'organe d'obturation 56. A cet effet, le corps 66 du piston intérieur 14 comporte un rebord annulaire 74 destiné à s'engager sur une protubérance frontale 75 ménagée le long de l'extrémité intérieure du bouchon 57. La fig. 11 illustre une position intermédiaire dans laquelle le piston intérieur est entrain d'adopter sa position finale.

Sur la fig. 12, l'obturateur 58 a été dévissé par rotation de la capsule 59 par rapport à l'ampoule 50, ce qui a pour effet de libérer le passage entre le tronçon d'extrémité tronconique 62 et le siège conique 63, passage communiquant avec le conduit 71 ménagé dans la soupape 15. Une simple pression sur le fond 72 de l'ampoule permet d'effectuer l'injection du mélange préparé dans la chambre 51.

Cette conception de seringue mélangeuse est particulièrement avantageuse en raison de la simplicité de sa réalisation et du faible nombre de pièces nécessaires à sa fabrication. Il en résulte un coût extrêmement compétitif ainsi que la possibilité d'automatiser entièrement le montage et le remplissage.

L'organe d'obturation utilisé dans la réalisation selon les figures 10 à 12 peut présenter diverses variantes illustrées notamment par les figures 13, 14 et 15, 16. L'organe d'obturation des figures 13 et 14 comporte un bouchon 80 dont la base 81 élargie est vissée à l'intérieur d'une capsule 82. Le bouchon 80 est pourvu d'un alésage central 83 dans lequel est engagé un obturateur 84 dont la paroi latérale est équipée d'une gorge axiale 85 communiquant avec le conduit central 86 d'un embout porte-aiguille 87. L'extrémité supérieure de l'obturateur 84 présente une forme tronconique qui coopère avec un siège conique constituant l'extrémité supérieure de l'alésage central 83 du bouchon 80 pour assurer l'étanchéité de l'ampoule et empêcher l'écoulement du liquide contenu dans la chambre inférieure à travers la gorge axiale 85.

Comme le montre la fig. 14, lorsque la capsule 82 est dévissée par rapport au bouchon 80, le tronçon d'extrémité tronconique de l'obturateur 84 est décalé vers le bas et permet l'évacuation du mélange dans le sens de la flèche H.

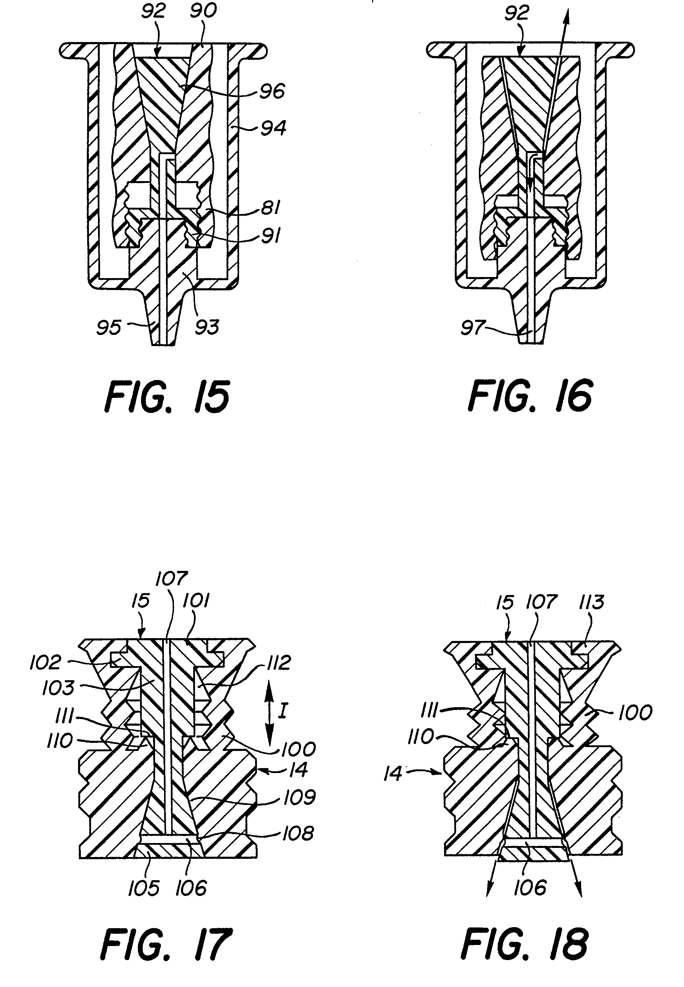

Les figures 15 et 16 représentent une autre variante comportant comme précédemment un bouchon 90 dont la base rétrécie 81 est pourvue d'un filetage permettant de visser ou de dévisser ce bouchon sur la base 91 d'un obturateur 92 logé dans un alésage central du bouchon 90. La base 91 de l'obturateur 92 est solidaire d'un bloc 93 réalisé d'une pièce avec la capsule 94 et l'embout porte-aiguille 95. L'obturateur 92 comporte un tronçon supérieur tronconique 96 dont la surface périphérique est, dans la position de stockage illustrée par la fig. 15, en appui contre les parois d'un siège conique qui constitue la partie supérieure de l'alésage central du bouchon 90. Comme précédemment, un déplacement relatif de l'obturateur 92 par rapport au bouchon 90 permet de libérer un passage suffisant pour assurer l'écoulement du mélange vers le conduit central 97 traversant le bloc 93 et le porte-aiguille 95.

Dans les réalisations illustrées par les figures 1 à 12, la soupape 15 du piston central était équipée d'organes ressorts logés dans une cavité intérieure au corps du piston. Cette conception selon laquelle les organes ressorts sont constitués par une pièce indépendante qu'il convient ensuite de monter dans la cavité intérieure du corps du piston, présente quelques inconvénients qui peuvent être résolus par les réalisations illustrées par les figures 17 à 22. Les figures 17 et 18 illustrent une première forme de réalisation d'un piston intérieur 14 muni d'une soupape 15 et d'organes ressorts 100 réalisés d'une pièce avec le corps du piston 14. Ces organes ressorts sont constitués par une structure annulaire dont la section axiale présente une forme plissée en accordéon et qui est réalisée en une matière élastomère souple susceptible de s'écraser dans le sens de la double flèche I. La soupape 15 comporte une partie supérieure 101 en contact avec la chambre contenant le liquide. Un rebord élargi 102 adjacent à la partie 101 permet de coupler la soupape au corps du piston 100. Un tronçon cylindrique 103 de section réduite assure la liaison entre la partie supérieure 101 et l'élément tronconique inférieur 105 orienté vers la chambre contenant la poudre surmontée d'un gaz compressible. Dans la position représentée par la fig. 17, le canal radial 106 communiquant avec le conduit axial 107 est obturé par un bourrelet annulaire 108 ménagé le long de la paroi du siège conique 109 dans lequel est logé l'élément tronconique 105 de la soupape 15. Un bourrelet annulaire 110 en forme de pointe sur laquelle prend appui un épaulement 111 du tronçon cylindrique 103 de la soupape 15, assure l'étanchéité de la cavité centrale 112 en empêchant une remontée de liquide le long des parois du siège conique 109.

Comme le montre la fig. 18, lorsqu'une pression est exercée sur la surface supérieure du piston et sur la surface annulaire adjacente du piston 14, les organes ressorts 100 s'écrasent ainsi que le bourrelet d'étanchéité 110, ce qui a pour effet de décaler axialement la partie tronconique 105 de la soupape 15 et son siège conique 109. Il en résulte un dégagement des orifices du conduit radial 106 et l'écoulement du liquide à travers ce conduit de la seconde chambre vers la première d'une manière qui a déjà été expliquée précédemment.

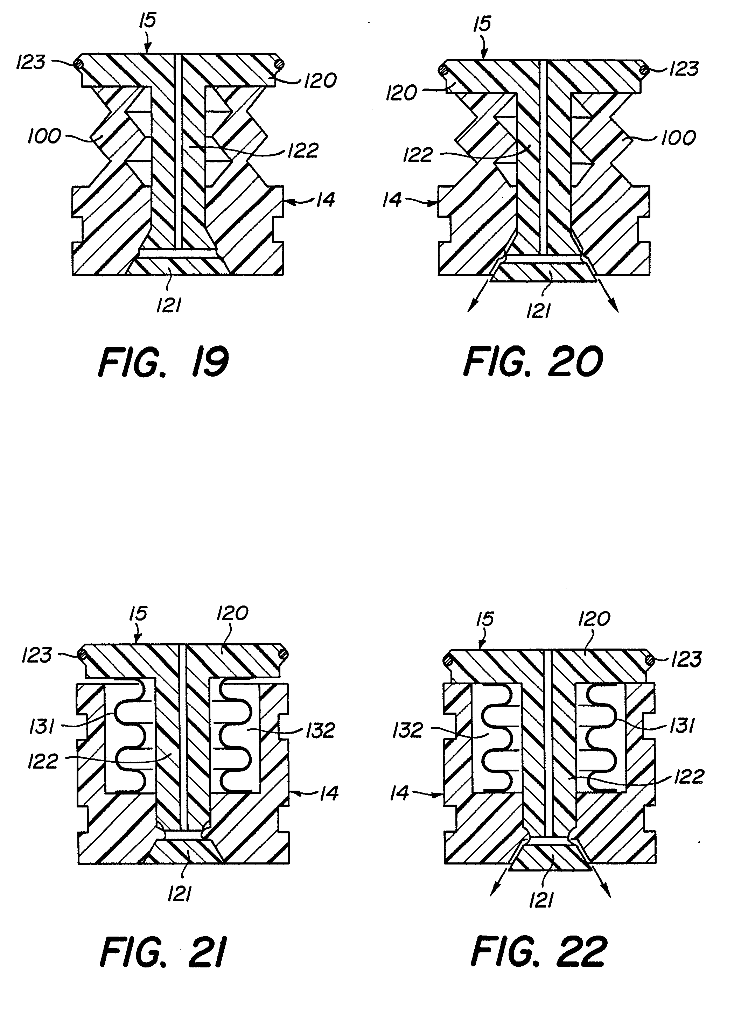

Les figures 19 et 20 illustrent une autre forme de réalisation du piston intérieur 14 et de sa soupape 15. Dans cet exemple la soupape 15 se compose d'une plaque supérieure 120 reliée à un embout tronconique 121 par un tronçon cylindrique 122. Comme la soupape 15 est de préférence réalisée en un matériau plus rigide que l'élastomère à l'aide duquel est réalisé le corps du piston 14, un joint d'étanchéité 123 est monté dans une gorge périphérique du plateau 120.

La réalisation illustrée par les figures 21 et 22 diffère de celle des figures 19 et 20 en ce que les organes ressorts 131 sont constitués par un ressort spirale métallique logé dans une cavité 132 ménagée à l'intérieur du corps du piston 14. Les autres éléments tels que la plaque supérieure 120 de la soupape 15, l'embout tronconique 121 et le tronçon cylindrique de liaison 122 ainsi que le joint d'étanchéité 123 sont identiques aux éléments correspondants du dispositif selon les figures 19 et 20.

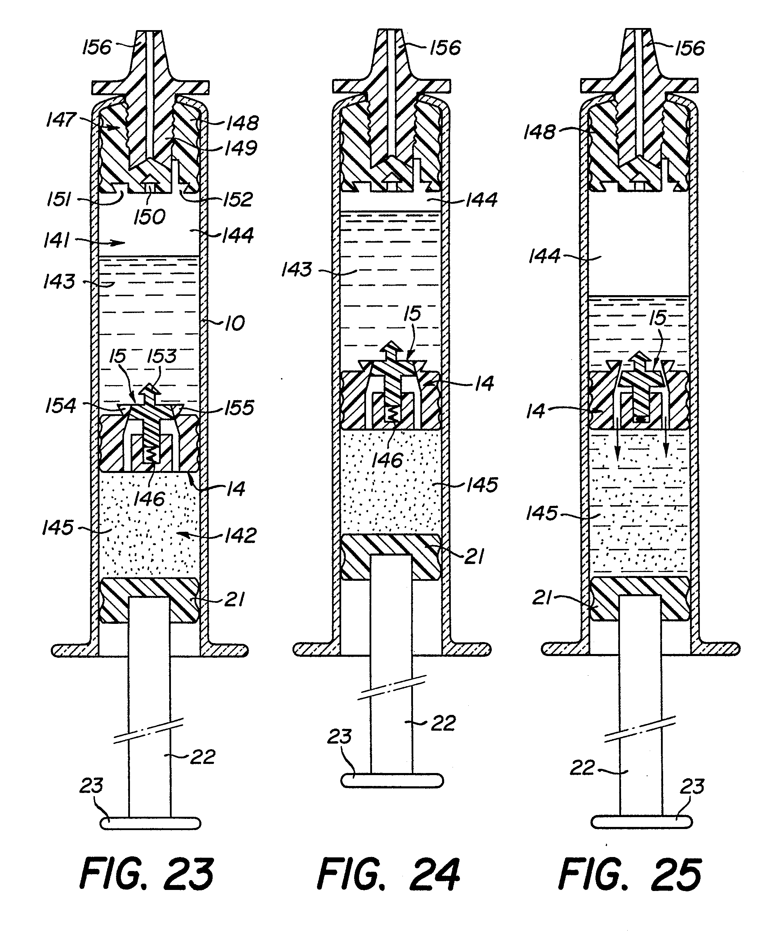

Alors que les systèmes précédents étaient totalement dépourvus d'organes de retenue, les seringues illustrées par les figures 23 à 26 comportent un piston intérieur pourvu d'organes de retenue puissants, mais qui, comme cela sera démontré par la suite, ne présentent aucun caractère gênant au moment de l'injection. Dans cette réalisation, le corps de seringue 10 est divisé en deux chambres 141 et 142 dont la première 141 contient un liquide 143 surmonté d'un gaz détendu 144, et dont la seconde 142 contient de préférence une poudre 145. Le piston intérieur 14 comporte une soupape 15 mobile axialement à l'intérieur d'un alésage central ménagé dans le corps du piston, et sollicitée vers sa position de fermeture par un ressort 146. Le corps de la seringue est obturé d'un côté par un piston extérieur 22 et de l'autre côté par un organe d'obturation 147 comportant un bouchon 148 disposé à l'intérieur du corps de la seringue et un obturateur 149 vissé dans un alésage du bouchon 148. La face intérieure du bouchon 148 présente des évidements 150, 151, 152 de formes complémentaires à celles des protubérances 153, 154 et 155 ménagées à la base du piston intérieur 14.

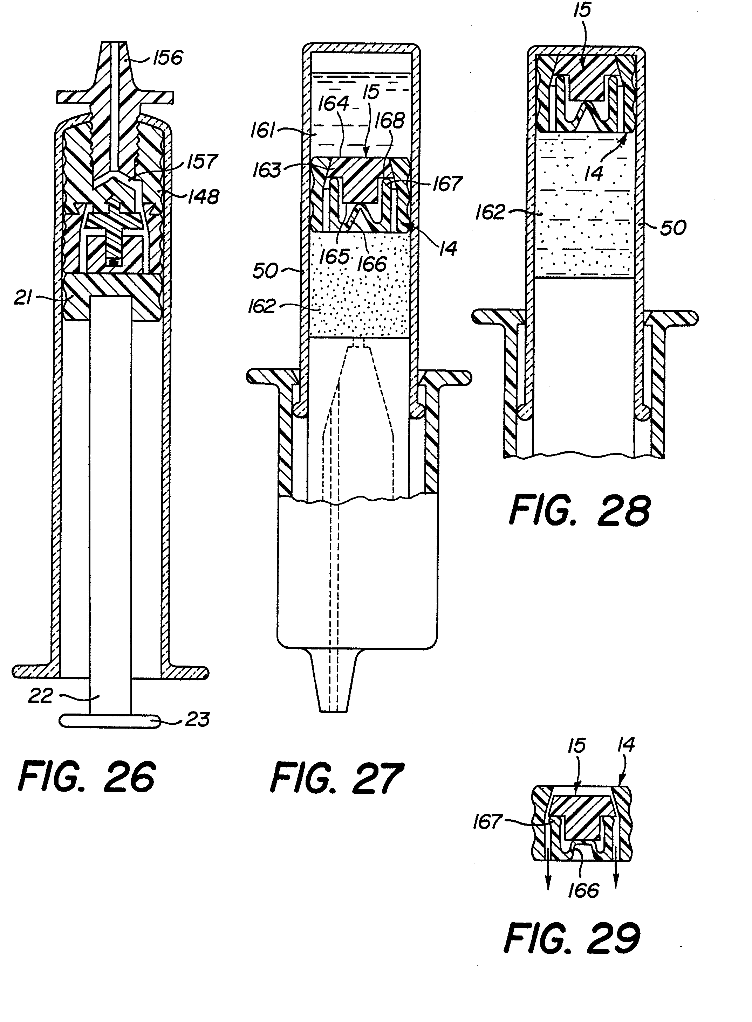

Dans cet exemple, le piston intérieur 14 comporte des organes de retenue puissants, localisés le long de sa périphérie, ces organes se présentant sous la forme de bourrelets élastiques prenant appui sur la paroi lisse du corps de la seringue. Lorsque l'opérateur exerce une poussée sur le piston extérieur 22, la poudre 145 est tout d'abord comprimée jusqu'à son stade d'incompressibilité qui est atteint rapidement. La pression exercée sur elle se transmet ensuite au piston intérieur, dont la soupape reste fermée puis au contenu de la chambre 141 dont le gaz 144 se comprime. Pendant toute cette phase de compression la soupape 14 reste fermée. Cette phase est illustrée par la fig. 24. Lorsqu'on atteint le stade d'incompressibilité du gaz 144, on relâche le piston 22, de telle manière qu'il se crée une pression différentielle entre les deux chambres. Sous l'effet de la surpression exercée par le gaz comprimé et en raison de l'existence des organes de retenue puissants qui maintiennent le piston intérieur en place, la soupape s'ouvre et au moins une partie du liquide 143 peut s'écouler vers la chambre 145 sans contre-pression ce qui permet une décharge complète et rapide. Lorsque le gaz est à nouveau détendu (voir fig. 24), la soupape se ferme sous l'effet de la poussée exercée par le ressort 146. Un nouveau cycle peut être enclenché. Lorsque tout le liquide 143 est passé dans la chambre 142, le piston intérieur 14 est entièrement refoulé au fond du corps de la seringue et les protubérances 153, 154 et 155 se sont engagées dans les évidements complémentaires 150, 151 et 152. Une rotation de l'obturateur 149 solidaire de la capsule 156 permet de dégager un passage 157 entre le bouchon 149 et l'obturateur 148 et d'assurer l'évacuation du mélange, l'obturateur 15 étant bloqué en position ouverte lorsque le piston intérieur et l'organe d'obturation sont accouplés (voir fig. 26).

L'ampoule-seringue illustrée par les figures 27 et 28 comporte une ampoule 50 identique à celle des figures 10 à 12 et munie d'un organe d'obturation similaire à l'organe d'obturation 56. Le piston intérieur 14 divise l'ampoule en deux chambres 161 et 162 dont la première contient de la poudre et l'autre un liquide et un gaz initialement détendu. Le dispositif fonctionne sensiblement comme le précédent. La poussée sur le fond de l'ampoule provoque une compression du gaz, la soupape 15 restant fermée. La détente du gaz, après relâchement de la pression, provoque l'ouverture de la soupape et le passage du liquide (voir fig. 29). La soupape 15 se compose d'un bloc tronconique 163 formant un plateau supérieur 164 et un plateau inférieur 165. Une butée centrale souple 166 prend appui sur le plateau inférieur 165, et une butée annulaire 167 prend appui sur un épaulement 168 ménagé entre le plateau supérieur et le plateau inférieur. Ces butées bloquent la soupape en position fermée sur les figures 27 et 28 et sont écrasées lorsque la soupape est ouverte, comme le montre la fig. 29.

Comme précédemment, le piston intérieur est refoulé dans une position dans laquelle les organes de retenue ne présentent aucune gêne à l'injection (voir fig. 28). Le dernier système notamment, est avantageux en ce que le nombre de pièces qui le composent est particulièrement réduit.

Il est bien entendu que la présente invention n'est pas limitée aux formes décrites mais peut subir diverses modifications évidentes pour l'homme de l'art.

Revendications

1. Seringue préremplie à double compartiment comportant un corps de seringue ainsi qu'un piston intérieur muni d'une soupape, et divisant le corps de la seringue en deux chambres contenant respectivement un produit liquide ou pulvérulent et un produit liquide destiné à être mélangé audit produit liquide ou pulvérulent à l'intérieur de la chambre correspondante, caractérisée en ce que le piston intérieur comporte un évidement central dans lequel est logée ladite soupape, en ce que cette soupape est mobile axialement dans cet évidement entre une première position dite position d'obturation et une seconde position dite positionn d'écoulement, en ce que la soupape présente une première surface d'appui orientée vers l'une des chambres et une seconde surface d'appui orientée vers l'autre chambre, et en ce que cette soupape comporte au moins un canal d'écoulement agencé pour faire communiquer les deux chambres entre elles pour permettre l'écoulement du liquide vers la chambre contenant ledit produit liquide ou pulvérulent lorsque la soupape se trouve dans sa position d'écoulement, ainsi que des organes ressorts pour solliciter cette soupape dans sa position d'obturation.

2. Seringue selon la revendication 1, caractérisée en ce que le corps de la seringue présente une surface intérieure lisse exempte de toute protubérance, de telle manière que le piston intérieur soit librement mobile selon une direction axiale.

3. Seringue selon la revendication 1, caractérisée en ce que la première surface d'appui de la soupape, orientée vers la chambre contenant le produit liquide à transvaser dans l'autre chambre contenant le produit liquide ou pulvérulent est supérieure à la seconde surface d'appui orientée vers ladite autre chambre.

4. Seringue selon la revendication 3, caractérisée en ce que la soupape comporte au moins un élément tronconique agencé pour prendre appui contre un siège conique ménagé dans le corps du piston intérieur, lorsque cette soupape se trouve dans ladite position d'obturation et pour ménager un passage entre lui et ce siège lorsque la soupape se trouve dans sa position d'écoulement.

5. Seringue selon la revendication 4, caractérisée en ce que l'élément tronconique de la soupape comporte ladtite seconde surface d'appui et est raccordé par un élément intermédiaire à un plateau comportant ladite première surface d'appui.

6. Seringue selon la revendication 1, caractérisée en ce que l'une des chambres contient un gaz inerte susceptible d'être comprimé pendant la phase de mélange des produits initialement contenus dans les deux chambres.

7. Seringue selon la revendication 6, caractérisée en ce que la chambre contenant le produit liquide à transvaser est localisée entre le piston intérieur et un piston extérieur fermant une extrémité du corps de la seringue, et en ce que l'autre chambre contenant le produit liquide ou pulvérulent, ainsi que le gaz susceptible d'être comprimé pendant la phase de mélange, est localisée entre un organe d'obturation fermant l'autre extrémité du corps de la seringue et le piston intérieur.

8. Seringue selon la revendication 6, caractérisée en ce que la chambre contenant le produit liquide à transvaser ainsi que le gaz susceptible d'être comprimé pendant la phase de mélange est localisée entre un organe d'obturation fermant l'une des extrémités du corps de la seringue et le piston intérieur et en ce que l'autre chambre contenant le produit liquide ou pulvérulent est localisée entre le piston intérieur et un piston extérieur fermant l'autre extrémité du corps de la seringue.

9. Seringue selon l'une quelconque des revendications 1 à 6, caractérisée en ce qu'elle comporte une ampoule fermée à une de ses extrémités.

10. Seringue selon la revendication 9, caractérisée en ce que la chambre contenant le produit liquide à transvaser est localisée entre l'organe d'obturation fermant l'ampoule à son extrémité ouverte et le piston intérieur, et en ce que la chambre contenant le produit liquide ou pulvérulent ainsi que le gaz susceptible d'être comprimé pendant la phase de mélange, est localisée entre le fond de l'ampoule et le piston intérieur.

11. Seringue selon la revendication 9, caractérisée en ce que la chambre contenant le produit liquide à transvaser ainsi que le gaz susceptible d'être comprimé pendant la phase de mélange est localisée entre le fond de l'ampoule et le piston intérieur, et en ce que l'autre chambre contenant le produit liquide ou pulvérulent est localisée entre l'organe d'obturation fermant l'extrémité ouverte de l'ampoule et le piston intérieur.

12. Seringue selon l'une quelconque des revendications précédentes, caractérisée en ce que le piston intérieur comporte des organes pour permettre son accrochage à un bouchon d'obturation du corps de la seringue.

13. Seringue selon la revendication 1, caractérisée en ce que les organes ressorts sont constitués par au moins un ressort logé dans un évidement ménagé à l'intérieur du corps du piston intérieur.

14. Seringue selon la revendication 1, caractérisée en ce que les organes ressorts sont réalisés d'une pièce avec le corps du piston intérieur, et comportent au moins un élément élastique reliant deux parties opposées du corps de ce piston.

15. Seringue selon l'une quelconque des revendications précédentes, caractérisée en ce l'organe d'obturation comporte des moyens d'accrochage agencés pour coopérer avec le piston intérieur et bloquer la soupape dans sa position d'écoulement.

FIG. 1          FIG. 2          FIG. 3

FIG. 4    FIG. 5    FIG. 6

FIG. 7      FIG. 8      FIG. 9

FIG. 10

FIG. 11

FIG. 12

FIG. 13

FIG. 14

FIG. 15

FIG. 16

FIG. 17

FIG. 18

0112574

FIG. 19

FIG. 20

FIG. 21

FIG. 22

FIG. 23          FIG. 24          FIG. 25

FIG. 26

FIG. 27

FIG. 28

FIG. 29

Office européen
des brevets

**RAPPORT DE RECHERCHE EUROPEENNE**

0112574
Numéro de la demande

EP 83 11 3098

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,X | FR-A-1 574 830 (H. WIMMER) <br> * page 6, ligne 25 - page 8, ligne 17; figures 1-10 * | 1-5,14 | A 61 M 5/28 <br> A 61 M 5/315 <br> B 65 D 81/32 |
| | --- | | |
| X | US-A-3 076 456 (P.B. HUNT) <br> * figures 1,4,5 * | 1-3,14 | |
| | --- | | |
| X | CH-A- 541 481 (M. LACROIX) <br> * figures 2,5 * | 1-3 | |
| A | | 13 | |
| | --- | | |
| X | DE-A-1 909 794 (Fa. ERHARDT SÖHNE) <br> * page 7, ligne 9 - page 8, ligne 11; figures 1-5 * | 1-3,14 | |
| | | | **DOMAINES TECHNIQUES RECHERCHES (Int. Cl. 3)** |
| Y | | 6-8,9-11,13 | A 61 M <br> A 61 J <br> B 65 D |
| | --- | | |
| Y | FR-A-2 082 128 (H.R. ROBERT) <br> * page 4, ligne 12 - page 5, ligne 9; figure 1 * | 6-8,13 | |
| | --- | | |
| Y | US-A-2 567 001 (T.E. WATSON) <br> * colonne 3, lignes 20-22; figures 1-4 * | 9-11 | |
| | --- -/- | | |

Le présent rapport de recherche a été établi pour toutes les revendications

| Lieu de la recherche <br> LA HAYE | Date d'achèvement de la recherche <br> 13-03-1984 | Examinateur <br> WOLF C.H.S. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

0112574
Numéro de la demande

EP 83 11 3098

| Catégorie | Citation du document avec indication, en cas de besoin, des parties pertinentes | Revendication concernée | CLASSEMENT DE LA DEMANDE (Int. Cl. ³) |
|---|---|---|---|
| D,A | US-A-3 380 451 (R.E. PORTER et al.)<br>* colonne 3, lignes 1-50; figures 2,3 * | 1,4,12,14,15 | |
| A | FR-A- 458 199 (P.A. GENTILE)<br>* en entier * | 4,13 | |
| A | FR-A-1 598 878 (ROUSSEL-UCLAF)<br>* page 3, ligne 23 - page 4, ligne 13; figures 1,3 * | 1-3,5 | |

### DOCUMENTS CONSIDERES COMME PERTINENTS

Page 2

DOMAINES TECHNIQUES RECHERCHES (Int. Cl. ³)

Le présent rapport de recherche a été établi pour toutes les revendications

| Lieu de la recherche<br>LA HAYE | Date d'achèvement de la recherche<br>13-03-1984 | Examinateur<br>WOLF C.H.S. |
|---|---|---|